# EUROPÄISCHE PATENTSCHRIFT

(11) **EP 0 738 715 B1**
(45) Veröffentlichungstag und Bekanntmachung des Hinweises auf die Patenterteilung: **29.01.2003**
(21) Anmeldenummer: 96105702.3
(22) Anmeldetag: 11.04.1996
(51) Int. Cl.: C07D 237/04, A61K 31/50

(54) **Arylalkyl-pyridazinone**
Arylalkyl-pyridazinones
Arylalkyl-pyridazinones

(30) Priorität: 20.04.1995 DE 19514568
(43) Veröffentlichungstag der Anmeldung: 23.10.1996
(73) Patentinhaber: MERCK PATENT GmbH, 64293 Darmstadt (DE)
(72) Erfinder: Jonas, Rochus, Dr., 64291 Darmstadt (DE); Wolf, Michael, Dr., 64297 Darmstadt (DE); Beier, Norbert, Dr., 64354 Reinheim (DE)

(56) Entgegenhaltungen:
- EP-A- 0 129 791
- EP-A- 0 175 363
- EP-A- 0 186 484
- EP-A- 0 279 283
- EP-A- 0 412 814
- EP-A- 0 667 158
- DE-A- 2 845 456
- DE-A- 19 502 699
- L. PITARCH ET AL.: EUR. J. MED. CHEM. - CHIMICA THERAPEUTICA, Bd. 9, Nr. 6, 1974, Seiten 644-50, XP000612184

## Beschreibung

Die Erfindung betrifft Verbindungen der Formel I 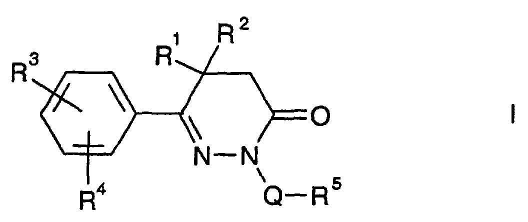 worin
- R¹: H,
- R²: Methyl oder Ethyl,
- R³ und R⁴: jeweils unabhängig voneinander -OH, -OR¹⁰, -S-R¹⁰, -SO-R¹⁰, -SO₂-R¹⁰, Hal, Methylendioxy, -NO₂, -NH₂, -NHR¹⁰ oder -NR¹⁰R¹¹,
- R⁵: einen ein- oder zweifach durch R⁶ und/oder R⁷ substituierten Phenylrest,
- Q: fehlt oder Alkylen mit 1-6 C-Atomen,
- R⁶ und R⁷: jeweils unabhängig voneinander -NH₂, -NR⁸R⁹, -NHR¹⁰, -NR¹⁰R¹¹, -NO₂, Hal, -CN, -OA, -COOH oder -COOA,
- R⁸ und R⁹: jeweils unabhängig voneinander H, Acyl mit 1-8 C-Atomen, das durch 1-5 F- und/oder Cl-Atome substituiert sein kann, -COOA, -SO-A, -SO₂A, -CONH₂, -CONHA, -CONA₂, -CO-COOH, -CO-COOA, -CO-CONH₂, -CO-CONHA oder -CO-CONA₂,
- A: Alkyl mit 1 bis 6 C-Atomen, das durch 1-5 F- und/oder Cl-Atome substituiert sein kann,
- R¹⁰ und R¹¹: jeweils unabhängig voneinander A, Cycloalkyl mit 3-7 C-Atomen, Methylencycloalkyl mit 4-8 C-Atomen oder Alkenyl mit 2-8 C-Atomen
und
- Hal: F, Cl, Br oder I
bedeuten,
sowie deren physiologisch unbedenklichen Salze.

Ähnliche Verbindungen sind aus DE 19502699.3 bekannt.

### < hier Seite 2a einfügen >

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit wertvolle pharmakologische Eigenschaften besitzen. Insbesondere zeigen sie Phosphodiesterase IV-Hemmung und können zur Behandlung von asthmatischen Erkrankungen eingesetzt werden. Die antiasthmatische Wirkung kann z.B. nach der Methode von T. Olsson, Acta allergologica 26, 438-447 (1971), bestimmt werden.

Die Verbindungen zeigen außerdem eine hemmende Wirkung auf die Bildung von TNF (Tumor Nekrose Faktor) und eignen sich daher zur Behandlung von allergischen und entzündlichen Krankheiten, Autoimmunkrankheiten und Transplantatabstoßungsreaktionen. Sie können zur Behandlung von Gedächtnisstörungen eingesetzt werden.

Die Verbindungen der Formel I können daher als Arzneimittelwirkstoffe in der Human- und Veterinärmedizin verwendet werden. Ferner können sie als Zwischenprodukte zur Herstellung weiterer Arzneimittelwirkstoffe eingesetzt werden.

Andere Pyridazinonderivate zur Behandlung von Herzschwäche sind in EP 0 412 814 A2 beschrieben, sowie in EP 0 667 158 A1 zur Verhinderung der Blutplättchenaggregation. In EP 0 279 283 A2 werden Pyridazinonderivate mit positiv inotroper Wirkung beschrieben. Aus DE 28 45 456 kennt man Pyridazinonderivate mit arteriosklerotischer und lipidspiegelsenkenden Eigenschaften. Andere Pyridazinone zur Behandlung von Erkrankungen des Herzens sind in EP 0 175 363 A2 und EP 0 129 791 A2 beschrieben. In EP 0 186 484 A2 sind Pyridazinonderivate zur Steigerung der Kontraktilität des Herzens offenbart. Andere Pyridazinonderivate mit anlagetischer und antiinflammatorischer Wirkung sind in Eur. J. Med. Chem. **9**, 644 (1974) beschrieben. In EP 0 618 201 A1 sind Thiadiazinonderivate zur Bekämpfung von cardiovaskulären sowie asthmatischen Erkrankungen beschrieben. PDE IV Inhibitoren, u.a. mit Pyridazinonstrukturen, sind in Drugs Future **17**, 799 (1992) und in Drugs News Perspect. **6**, 203 (1993) beschrieben.

Gegenstand der Erfindung sind dementsprechend die Verbindungen der Formel I und ihre Salze sowie ein Verfahren zur Herstellung dieser Verbindungen sowie ihrer Salze, dadurch gekennzeichnet, daß man eine Verbindung der Formel II 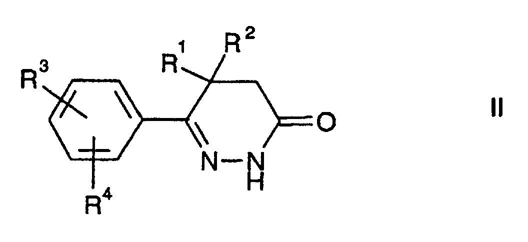 worin
R¹, R², R³ und R⁴ die in Anspruch 1 angegebenen Bedeutungen haben,
mit einer Verbindung der Formel III

R⁵-Q-X III

worin
- R⁵ und Q: die angegebenen Bedeutungen haben, und
- X: Cl, Br, OH oder eine reaktionsfähige veresterte OH-Gruppe bedeutet,
umsetzt,
oder daß man eine Verbindung der Formel IV 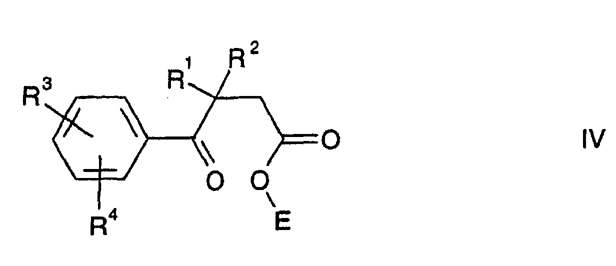 worin
- R¹, R², R³ und R⁴: die angegebenen Bedeutungen haben, und
- E: H oder Alkyl mit 1-4 C-Atomen bedeutet,
mit einer Verbindung der Formel V

H₂N-NH-Q-R⁵ V

worin
Q und R⁵ die angegebenen Bedeutungen haben,
umsetzt,
oder daß man in einer Verbindung der Formel I einen Rest R⁵ in einen anderen Rest R⁵ umwandelt, indem man eine Nitrogruppe reduziert, eine primäre oder eine sekundäre Aminogruppe alkyliert oder acyliert oder eine Cyangruppe hydrolysiert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ und X die angegebenen Bedeutungen haben, umsetzt und/oder eine Base der Formel I durch Behandeln mit einer Säure in eines ihrer Salze umwandelt.

Vor- und nachstehend haben die Reste R¹, R², R³, R⁴, R⁵, Q und X die bei den Formeln I, II und III angegebenen Bedeutungen, falls nicht ausdrücklich etwas anderes angegeben ist.

A bedeutet Alkyl.

In den vorstehenden Formeln ist Alkyl vorzugsweise unverzweigt und hat 1 bis 6 C-Atome, vorzugsweise 1, 2, 3 oder 4 C-Atome und bedeutet vorzugsweise Methyl, ferner bevorzugt Ethyl oder Propyl, weiterhin bevorzugt Isopropyl, Butyl, Isobutyl, sek.-Butyl oder tert.-Butyl, aber auch n-Pentyl oder Isopentyl.

Cycloalkyl hat vorzugsweise 3-7 C-Atome und steht bevorzugt für Cyclopropyl oder Cyclobutyl, weiterhin bevorzugt für Cyclopentyl oder Cyclohexyl, ferner auch für Cycloheptyl.

Methylencycloalkyl hat vorzugsweise 4-8 C-Atome und steht bevorzugt für Methylencyclopropyl und Methylencyclobutyl, weiterhin bevorzugt für Methylencyclopentyl und Methylencyclohexyl, ferner auch für Methylencycloheptyl.

Alkenyl steht vorzugsweise für Vinyl, 1- oder 2-Propenyl, 1-Butenyl, Isobutenyl, sek.-Butenyl, ferner bevorzugt ist 1-Pentenyl, iso-Pentenyl oder 1-Hexenyl.

Alkylen ist vorzugsweise unverzweigt und bedeutet bevorzugt Methylen oder Ethylen, ferner bevorzugt Propylen oder Butylen.

Von den Resten R¹ und R² steht einer vorzugsweise für H, während der andere bevorzugt Ethyl oder Methyl bedeutet.

Hal bedeutet vorzugsweise F, Cl oder Br, aber auch I.

Die Reste R³ und R⁴ können gleich oder verschieden sein und stehen vorzugsweise in der 3- oder 4-Position des Phenylrings. Sie bedeuten beispielsweise unabhängig voneinander Hydroxy, -S-CH₃, -SO-CH₃, -SO₂CH₃, F, Cl, Br oder I oder zusammen Methylendioxy. Besonders bevorzugt stehen sie aber jeweils für Methoxy, Ethoxy, Propoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.

Der Rest R⁵ bedeutet vorzugsweise ein- oder zweifach substituiertes Phenyl. Bevorzugte Substituenten sind Cyan, Nitro, Amino, Acetamido, Trifluoracetamido, Methoxy und/oder Chlor, ferner sind bevorzugt Methylsulfonamido, Propionylamino, 2-Methylpropionylamino, Isobutyrylamino und /oder Pivalylamino, weiter bevorzugt sind Methoxycarbonylamino, Methoxalylamino, Ureido und/oder Carboxy.

Q-R⁵ ist vorzugsweise Benzyl, 2-, 3- oder 4-Nitrobenzyl, 2-, 3- oder 4-Cyanbenzyl, 2-, 3- oder 4-Aminobenzyl, 2-, 3- oder 4-Acetamidobenzyl, 2-, 3- oder 4-Trifluoracetamidobenzyl, 2-, 3- oder 4-Methoxybenzyl, 2-, 3- oder 4-Chlorbenzyl, weiter bevorzugt ist 2-, 3- oder 4-Methylsulfonamido-benzyl, 2-, 3- oder 4-Propionylaminobenzyl, 2-, 3- oder 4-(2-Methylpropionylamino)-benzyl, 2, 3- oder 4-Isobutyrylaminobenzyl, 2-, 3- oder 4-Pivalylaminobenzyl, 2-, 3- oder 4-Methoxycarbonylaminobenzyl, 2-, 3- oder 4-Ureidobenzyl, 2-, 3- oder 4-Carboxybenzyl, 2-, 3- oder 4-Methoxalylaminobenzyl, ferner bevorzugt ist 2,3-, 2,4-, 2,5-, 2,6-, 3,4- oder 3,5-Dinitrobenzyl, 2,3-, 2,4-, 2,5-, 2,6-, 3,4- oder 3,5-Diaminobenzyl, 2,3-, 2,4-, 2,5-, 2,6-, 3,4- oder 3,5-Diacetamidobenzyl, 2,3-, 2,4-, 2,5-, 2,6-, 3,4- oder 3,5-Bis-(trifluoracetamido)-benzyl, 2,3-, 2,4-, 2,5-, 2,6-, 3,4- oder 3,5-Dimethoxybenzyl, 2,3-, 2,4-, 2,5-, 2,6-, 3,4- oder 3,5-Dichlorbenzyl, 2,3-, 2,4-, 2,5-, 2,6-, 3,4- oder 3,5-Dimethylsulfonamidobenzyl, 2,3-, 2,4-, 2,5-, 2,6-, 3,4- oder 3,5-Dipropionylaminobenzyl, 2,3-, 2,4-, 2,5-, 2,6-, 3,4- oder 3,5-Bis-(2-Methylpropionylamino)-benzyl, 2,3-, 2,4-, 2,5-, 2,6-, 3,4- oder 3,5-Diisobutyrylaminobenzyl, 2,3-, 2,4-, 2,5-, 2,6-, 3,4- oder 3,5-Dimethoxycarbonylaminobenzyl, 2,3-, 2,4-, 2,5-, 2,6-, 3,4- oder 3,5-Dimethoxalylaminobenzyl, 2,3-, 2,4-, 2,5-, 2,6-, 3,4- oder 3,5-Diureidobenzyl, 2,3-, 2,4-, 2,5-, 2,6-, 3,4- oder 3,5-Dicarboxybenzyl.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ic ausgedrückt werden, die der Formel I entsprechen und worin die nicht näher bezeichneten Reste die bei der Formel I angegebene Bedeutung haben, worin jedoch
in la
   - R¹: H,
   - R²: Methyl oder Ethyl,
   - R³ und R⁴: jeweils unabhängig voneinander OA
   bedeuten;
in Ib
   - R¹: H,
   - R²: Methyl oder Ethyl,
   - R³: OA
   - R⁴: mono-, di- oder trifluorsubstituiertes Alkyl mit 1 bis 6 C-Atomen
   bedeuten;
in Ic
   - R¹: H,
   - R²: Methyl oder Ethyl,
   - R³ und R⁴: jeweils unabhängig voneinander OR¹⁰,
   - R⁵: einen ein- oder zweifach substituierten Phenylrest
   bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der DE 195502699.3), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II und IV haben R¹, R², R³ und R⁴ die angegebenen Bedeutungen, insbesondere die angegebenen bevorzugten Bedeutungen.

In den Verbindungen der Formeln III und V steht Q vorzugsweise für Methylen oder Ethylen, ferner bevorzugt für Propylen oder Butylen.

In den Verbindungen der Formel IV steht E vorzugsweise für H, Methyl oder Ethyl, ferner auch für Propyl oder Butyl.

R⁵ hat in den Verbindungen der Formeln III und V die angegebenen bevorzugten Bedeutungen, während X Cl, Br, OH oder eine reaktionsfähige veresterte OH-Gruppe bedeutet.

Falls X eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyloder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt. Sofern sie nicht bekannt sind, können sie nach an sich bekannten Methoden hergestellt werden.

Pyridazinone der Formel II sind z.B. in Eur. J. Med. Chem. - Chim. Therapeut. 9, 644-650 (1977) beschrieben.

Die Verbindungen der Formel III werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Im einzelnen erfolgt die Umsetzung der 2,3,4,5-Tetrahydro-pyridazinone mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Die Verbindungen der Formel I können weiterhin erhalten werden, indem man Verbindungen der Formel IV mit Verbindungen der Formel V umsetzt.

Im einzelnen erfolgt die Umsetzung der Verbindungen der Formeln IV und V in Gegenwart oder Abwesenheit eines inerten Lösungsmittels und bei Temperaturen wie oben beschrieben.

Die Ausgangsstoffe der Formeln IV und V sind teilweise bekannt. Sofern sie nicht bekannt sind, können sie nach an sich bekannten Methoden hergestellt werden.

Die Verbindungen der Formeln IV und V werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Es ist ferner möglich, in einer Verbindung der Formel I einen Rest R⁵ in einen anderen Rest R⁵ umzuwandeln, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert oder Cyangruppen zu COOH-Gruppen hydrolysiert. Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

Ebenso ist es möglich, eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ sowie X die angegebenen Bedeutungen haben, umzusetzen. Die Veretherung der OH-Gruppen erfolgt nach an sich bekannten Methoden, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können, falls gewünscht, die freien Basen der Formel I aus ihren Salzen mit Basen (z.B. Natrium- oder Kaliumhydroxid oder - carbonat) in Freiheit gesetzt werden.

Verbindungen der Formel I können eine oder mehrere Asymmetriezentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch-aktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Die Formel I umschließt alle Stereoisomeren und deren Gemische, z.B. die Racemate.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase IV-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungsund/Oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cAMP(cyclo-Adenosin-monophosphät)-Spiegels zu Entzündungshemmung oder -Verhinderung und Muskelentspannung führt, eingesetzt werden. Besondere Verwendung können die erfindungsgemäßen Verbindungen bei der Behandlung von Allergien, Asthma, chronischer Bronchitis, atopischer Dermatitis, Psoriasis und anderer Hautkrankheiten und Autoimmunerkrankungen finden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

### Beispiel 1

Eine Suspension von 4,70 g 6-(3,4-Dimethoxyphenyl)-2,3,4,5-tetrahydropyridazin-3-on ("A") in 150 ml THF wird mit 2,24 g Kalium-tert.-butylat versetzt und 30 Minuten gerührt. Man gibt 4,32 g 4-Nitrobenzylchlorid dazu und rührt 10 Stunden bei Raumtemperatur nach. Das Lösungsmittel wird entfernt und wie üblich aufgearbeitet. Man erhält 2-(4-Nitrobenzyl)-6-(3,4-dimethoxyphenyl)-2,3,4,5-tetrahydro-pyridazin-3-on, F. 126°.

### Beispiel 2

Analog Beispiel 1 erhält man durch Umsetzung von 6-(3,4-Dimethoxyphenyl)-5-ethyl-2,3,4,5-tetrahydro-pyridazin-3-on ("B") mit 4-Nitrobenzylchlorid das 2-(4-Nitrobenzyl)-6-(3,4-dimethoxyphenyl)-5-ethyl-2,3,4,5-tetrahydro-pyridazin-3-on.

Analog erhält man durch Umsetzung von "B"
mit 3-Nitrobenzylchlorid:
2-(3-Nitrobenzyl)-6-(3,4-dimethoxyphenyl)-5-ethyl-2,3,4,5-tetrahydropyridazin-3-on;
mit 2-Nitrobenzylchlorid:
2-(2-Nitrobenzyl)-6-(3,4-dimethoxyphenyl)-5-ethyl-2,3,4,5-tetrahydropyridazin-3-on;
mit 2,3-Dinitrobenzylchlorid:
2-(2,3-Dinitrobenzyl)-6-(3,4-dimethoxyphenyl)-5-ethyl-2,3,4,5-tetrahydro-pyridazin-3-on;
mit 2,4-Dinitrobenzylchlorid:
2-(2,4-Dinitrobenzyl)-6-(3,4-dimethoxyphenyl)-5-ethyl-2,3,4,5-tetrahydro-pyridazin-3-on;
mit 2-Methoxybenzylchlorid:
2-(2-Methoxybenzyl)-6-(3,4-dimethoxyphenyl)-5-ethyl-2,3,4,5-tetrahydro-pyridazin-3-on;
mit 4-Methoxybenzylchlorid:
2-(4-Methoxybenzyl)-6-(3,4-dimethoxyphenyl)-5-ethyl-2,3,4,5-tetrahydro-pyridazin-3-on;
mit 2-Chlorbenzylchlorid:
2-(2-Chlorbenzyl)-6-(3,4-dimethoxyphenyl)-5-ethyl-2,3,4,5-tetrahydropyridazin-3-on;
mit 2,6-Dichlorbenzylchlorid:
2-(2,6-Dichlorbenzyl)-6-(3,4-dimethoxyphenyl)-5-ethyl-2,3,4,5-tetrahydro-pyridazin-3-on;
mit 4-Cyanbenzylchlorid:
2-(4-Cyanbenzyl)-6-(3,4-dimethoxyphenyl)-5-ethyl-2,3,4,5-tetrahydropyridazin-3-on;
mit 4-Carboxybenzylchlorid:
2-(4-Carboxybenzyl)-6-(3,4-dimethoxyphenyl)-5-ethyl-2,3,4,5-tetrahydro-pyridazin-3-on.

### Beispiel 3

Analog Beispiel 1 erhält man durch Umsetzung von 6-(3-Methoxy-4-trifluormethoxyphenyl)-5-ethyl-2,3,4,5-tetrahydro-pyridazin-3-on ("B") mit 4-Nitrobenzylchlorid ("C") das 2-(4-Nitrobenzyl)-6-(3-methoxy-4-trifluormethoxyphenyl)-5-ethyl-2,3,4,5-tetrahydro-pyridazin-3-on.

Analog erhält man durch Umsetzung von "C"
mit 6-(3-Methoxy-4-difluormethoxyphenyl)-5-ethyl-2,3,4,5-tetrahydropyridazin-3-on:
2-(4-Nitrobenzyl)-6-(3-methoxy-4-difluormethoxyphenyl)-5-ethyl-2,3,4,5-tetrahydro-pyridazin-3-on;
mit 6-(3-Methoxy-4-fluormethoxyphenyl)-5-ethyl-2,3,4,5-tetrahydropyridazin-3-on:
2-(4-Nitrobenzyl)-6-(3-methoxy-4-fluormethoxyphenyl)-5-ethyl-2,3,4,5-tetrahydro-pyridazin-3-on;
mit 6-(3-Difluormethoxy-4-methoxyphenyl)-5-ethyl-2,3,4,5-tetrahydropyridazin-3-on:
2-(4-Nitrobenzyl)-6-(3-difluormethoxy-4-methoxyphenyl)-5-ethyl-2,3,4,5-tetrahydro-pyridazin-3-on;
mit 6-(3-Trifluormethoxy-4-methoxyphenyl)-5-ethyl-2,3,4,5-tetrahydropyridazin-3-on:
2-(4-Nitrobenzyl)-6-(3-trifluormethoxy-4-methoxyphenyl)-5-ethyl-2,3,4,5-tetrahydro-pyridazin-3-on;
mit 6-(3-Fluormethoxy-4-methoxyphenyl)-5-ethyl-2,3,4,5-tetrahydropyridazin-3-on:
2-(4-Nitrobenzyl)-6-(3-Fluormethoxy-4-methoxyphenyl)-5-ethyl-2,3,4,5-tetrahydro-pyridazin-3-on;
mit 6-(3-Methoxy-4-ethoxyphenyl)-5-ethyl-2,3,4,5-tetrahydro-pyridazin-3-on:
2-(4-Nitrobenzyl)-6-(3-Methoxy-4-ethoxyphenyl)-5-ethyl-2,3,4,5-tetrahydro-pyridazin-3-on;
mit 6-(3-Ethoxy-4-methoxyphenyl)-5-ethyl-2,3,4,5-tetrahydro-pyridazin-3-on:
2-(4-Nitrobenzyl)-6-(3-Ethoxy-4-methoxyphenyl)-5-ethyl-2,3,4,5-tetrahydro-pyridazin-3-on;
mit 6-(3-Hydroxy-4-methoxyphenyl)-5-ethyl-2,3,4,5-tetrahydro-pyridazin-3-on:
2-(4-Nitrobenzyl)-6-(3-hydroxy-4-methoxyphenyl)-5-ethyl-2,3,4,5-tetrahydro-pyridazin-3-on;
mit 6-(4-Methylsulfonylphenyl)-5-ethyl-2,3,4,5-tetrahydro-pyridazin-3-on:
2-(4-Nitrobenzyl)-6-(4-methylsulfonylyphenyl)-5-ethyl-2,3,4,5-tetrahydro-pyridazin-3-on;
mit 6-(4-Methylenoxyphenyl)-5-ethyl-2,3,4,5-tetrahydro-pyridazin-3-on:
2-(4-Nitrobenzyl)-6-(4-methylenoxyphenyl)-5-ethyl-2,3,4,5-tetra- hydropyridazin-3-on;
mit 6-(3-Cyclopentyloxy-4-methoxy)-5-ethyl-2,3,4,5-tetrahydro-pyridazin-3-on:
2-(4-Nitrobenzyl)-6-(3-cyclopentyloxy-4-methoxyphenyl)-5-ethyl-2,3,4,5-tetrahydro-pyridazin-3-on.

### Beispiel 4

Eine Lösung von 4,6 g 2-(4-Nitrobenzyl)-6-(3,4-dimethoxyphenyl)-2,3,4,5-tetrahydro-pyridazin-3-on in 60 ml Methanol wird in Gegenwart von Raney-Nickel hydriert. Der Katalysator wird abfiltriert und die Lösung eingeengt. Man erhält nach Umkristallisation 2-(4-Aminobenzyl)-6-(3,4-dimethoxyphenyl)-2,3,4,5-tetrahydropyridazin-3-on, F. 184°.

Analog erhält man durch Hydrierung
von 2-(4-Nitrobenzyl)-6-(3,4-dimethoxyphenyl)-5-ethyl-2,3,4,5-tetrahydropyridazin-3-on:
2-(4-Aminobenzyl)-6-(3,4-dimethoxyphenyl)-5-ethyl-2,3,4,5-tetrahydropyridazin-3-on;
von 2-(3-Nitrobenzyl)-6-(3,4-dimethoxyphenyl)-5-ethyl-2,3,4,5-tetrahydropyridazin-3-on:
2-(3-Aminobenzyl)-6-(3,4-dimethoxyphenyl)-5-ethyl-2,3,4,5-tetrahydropyridazin-3-on, F. 49°;
von 2-(2-Nitrobenzyl)-6-(3,4-dimethoxyphenyl)-5-ethyl-2,3,4,5-tetrahydropyridazin-3-on:
2-(2-Aminobenzyl)-6-(3,4-dimethoxyphenyl)-5-ethyl-2,3,4,5-tetrahydropyridazin-3-on;
von 2-(2,3-Dinitrobenzyl)-6-(3,4-dimethoxyphenyl)-5-ethyl-2,3,4,5-tetrahydro-pyridazin-3-on:
2-(2,3-Diaminobenzyl)-6-(3,4-dimethoxyphenyl)-5-ethyl-2,3,4,5-tetrahydro-pyridazin-3-on;
von 2-(2,4-Dinitrobenzyl)-6-(3,4-dimethoxyphenyl)-5-ethyl-2,3,4,5-tetrahydro-pyridazin-3-on:
2-(2,4-Diaminobenzyl)-6-(3,4-dimethoxyphenyl)-5-ethyl-2,3,4,5-tetrahydro-pyridazin-3-on;
von 2-(4-Nitrobenzyl)-6-(3-methoxy-4-trifluormethoxyphenyl)-5-ethyl-2,3,4,5-tetrahydro-pyridazin-3-on:
2-(4-Aminobenzyl)-6-(3-methoxy-4-trifluormethoxyphenyl)-5-ethyl-2,3,4,5-tetrahydro-pyridazin-3-on;
von 2-(4-Nitrobenzyl)-6-(3-methoxy-4-dlfluormethoxyphenyl)-5-ethyl-2,3,4,5-tetrahydro-pyridazin-3-on:
2-(4-Aminobenzyl)-6-(3-methoxy-4-difluormethoxyphenyl)-5-ethyl-2,3,4, 5-tetrahydro-pyridazin-3-on;
von 2-(4-Nitrobenzyl)-6-(3-methoxy-4-fluormethoxyphenyl)-5-ethyl-2,3,4,5-tetrahydro-pyridazin-3-on:
2-(4-Aminobenzyl)-6-(3-methoxy-4-fluormethoxyphenyl)-5-ethyl-2,3,4,5-tetrahydro-pyridazin-3-on;
von 2-(4-Nitrobenzyl)-6-(3-difluormethoxy-4-methoxyphenyl)-5-ethyl-2,3,4,5- tetrahydro-pyridazin-3-on:
2-(4-Aminobenzyl)-6-(3-difluormethoxy-4-methoxyphenyl)-5-ethyl-2,3,4,5-tetrahydro-pyridazin-3-on;
von 2-(4-Nitrobenzyl)-6-(3-trifluormethoxy-4-methoxyphenyl)-5-ethyl-2,3,4,5-tetrahydro-pyridazin-3-on:
2-(4-Aminobenzyl)-6-(3-trifluormethoxy-4-methoxyphenyl)-5-ethyl-2,3,4,5-tetrahydro-pyridazin-3-on;
von 2-(4-Nitrobenzyl)-6-(3-Fluormethoxy-4-methoxyphenyl)-5-ethyl-2,3,4,5-tetrahydro-pyridazin-3-on:
2-(4-Aminobenzyl)-6-(3-Flormethoxy-4-methoxyphenyl)-5-ethyl-2,3,4,5-tetrahydro-pyridazin-3-on;
von 2-(4-Nitrobenzyl)-6-(3-Methoxy-4-ethoxyphenyl)-5-ethyl-2,3,4,5-tetrahydro-pyridazin-3-on:
2-(4-Aminobenzyl)-6-(3-Methoxy-4-ethoxyphenyl)-5-ethyl-2,3,4,5-tetrahydro-pyridazin-3-on;
von 2-(4-Nitrobenzyl)-6-(3-Ethoxy-4-methoxyphenyl)-5-ethyl-2,3,4,5-tetrahydro-pyridazin-3-on:
2-(4-Aminobenzyl)-6-(3-Ethoxy-4-methoxyphenyl)-5-ethyl-2,3,4,5-tetrahydro-pyridazin-3-on;
von 2-(4-Nitrobenzyl)-6-(3-hydroxy-4-methoxyphenyl)-5-ethyl-2,3,4,5-tetrahydro-pyridazin-3-on:
2-(4-Aminobenzyt)-6-(3-hydroxy-4-methoxyphenyl)-5-ethyl-2,3,4,5-tetrahydro-pyridazin-3-on;
von 2-(4-Nltrobenzyl)-6-(4-methylsuffonylphenyl)-5-ethyl-2,3,4,5-tetrahydro-pyridazin-3-on:
2-(4-Aminobenzyl)-6-(4-methylsulfonylphenyl)-5-ethyl-2,3,4,5-tetrahydro-pyridazin-3-on;
von 2-(4-Nitrobenzyl)-6-(4-methylenoxyphenyl)-5-ethyl-2,3,4,5-tetra-hydropyridazin-3-on:
2-(4-Aminobenzyl)-6-(4-methylenoxyphenyl)-5-ethyl-2,3,4,5-tetra-hydropyridazin-3-on;
von 2-(4-Nltrobenzyl)-6-(3-cyclopentyloxy-4-methoxyphenyl)-5-ethyl-2,3,4,5- tetrahydro-pyridazin-3-on:
2-(4-Aminobenzyl)-6-(3-cyclopentyloxy-4-methoxyphenyl)-5-ethyl-2,3,4,5-tetrahydro-pyridazin-3-on;
von 2-(3-Nitrobenzyl)-6-(3-cyctopentytoxy-4-methoxyphenyl)-5-ethyl-2,3,4,5-tetrahydro-pyridazin-3-on:
2-(3-Aminobenzyl)-6-(3-cyclopentyloxy-4-methoxyphenyl)-5-ethyl-2,3,4,5-tetrahydro-pyridazin-3-on, F. 109°;
von 2-(4-Nitrophenethyl)-6-(3,4-dimethoxyphenyl)-5-ethyl-2,3,4,5-tetrahydro-pyridazin-3-on:
2-(4-Aminophenethyl)-6-(3,4-dimethoxyphenyl)-5-ethyl-2,3,4,5-tetrahydro-pyridazin-3-on.

### Beispiel 5

Zu einer gekühlten Lösung von 1,2 g NaOH in 100 ml Wasser wird unter Rühren 10 g 2-(4-Cyanbenzyl)-6-(3,4-dihydroxyphenyl)-5-ethyl-2,3,4,5-tetrahydro-pyridazin-3-on gegeben und 10 Stunden nachgerührt. Man erwärmt vorsichtig und leitet einen Luftstrom durch die Lösung. Anschliessend wird gekühlte Schwefelsäure und Wasser zugegeben. Man arbeitet wie üblich auf und erhält 2-(4-Carboxybenzyl)-6-(3,4-dihydroxyphenyl)-5-ethyl-2,3,4,5-tetrahydro-pyridazin-3-on.

### Beispiel 6

Eine Lösung von 3,0 g 2-(4-Aminobenzyl)-6-(3,4-dimethoxyphenyl)-2,3,4,5-tetrahydro-pyridazin-3-on ("D") und 0,75 ml Pyridin in 80 ml Dichlormethan wird mit 1,0 g Buttersäurechlorid versetzt und eine Stunde nachgerührt. Man entfernt das Lösungsmittel und arbeitet wie üblich auf. Nach Umkristallisation erhält man 2-(4-Butyrylaminobenzyl)-6-(3,4-dimethoxyphenyl)-2,3,4,5-tetrahydro-pyridazin-3-on, F. 148°.

Analog erhält man durch Umsetzung von 2-(4-Aminobenzyl)-6-(3,4-dimethoxyphenyl)-5-ethyl-2,3,4,5-tetrahydro-pyridazin-3-on
mit Acetylchlorid:
2-(4-Acetamidobenzyl)-6-(3,4-dimethoxyphenyl)-5-ethyl-2,3,4,5-tetrahydropyridazin-3-on;
mit Trifluoracetylchlorid:
2-(4-Trifluoracetamidobenzyl)-6-(3,4-dimethoxyphenyl)-5-ethyl-2,3,4,5-tetrahydropyridazin-3-on, F. 133°;
mit Methylsulfonylchlorid:
2-(4-Methylsulfonamidobenzyl)-6-(3,4-dimethoxyphenyl)-5-ethyl-2,3,4,5-tetrahydropyridazin-3-on;
mit Propionylchiorid:
2-(4-Propionylaminobenzyl)-6-(3,4-dimethoxyphenyl)-5-ethyl-2,3,4,5-tetrahydropyridazin-3-on, F. 81°;
mit 2,2-Dimethylpropionylchlorid:
2-(4-tert.-Butylcarbonylaminobenzyl)-6-(3,4-dimethoxyphenyl)-5-ethyl-2,3,4,5-tetrahydropyridazin-3-on;
mit Butyrylchlorid:
2-(4-Butyrylaminobenzyl)-6-(3,4-dimethoxyphenyl)-5-ethyl-2,3,4,5-tetrahydropyridazin-3-on, F. 117°;
mit Isobutyrylchlorid:
2-(4-lsobutyrylaminobenzyl)-6-(3,4-dimethoxyphenyl)-5-ethyl-2,3,4,5-tetrahydropyridazin-3-on;
mit Chlorameisensäuremethylester:
2-(4-Methoxycarbonylaminobenzyl)-6-(3,4-dimethoxyphenyl)-5-ethyl-2,3,4,5-tetrahydropyridazin-3-on, F. 144°;
mit Pivalylchlorid:
2-(4-Pivalylaminobenzyl)-6-(3,4-dimethoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3,4-dimethoxyphenyl)-5-ethyl-2,3,4,5-tetrahydropyridazin-3-on;
mit Chlorameisensäureethylester:
2-(4-Ethoxycarbonylaminobenzyl)-6-(3,4-dimethoxyphenyl)-5-ethyl-2,3,4,5-tetrahydropyridazin-3-on, F.154°;
mit Methoxalylchlorid:
2-(4-Methoxalylaminobenzyl)-6-(3,4-dimethoxyphenyl)-5-ethyl-2,3,4,5-tetrahydropyridazin-3-on;
mit Chlorformamid:
2-(4-Ureidobenzyl)-6-(3,4-dimethoxyphenyl)-5-ethyl-2,3,4,5-tetrahydropyridazin-3-on;
mit Pentanoylchlorid:
2-(4-Pentanoylaminobenzyl)-6-(3,4-dimethoxyphenyl)-5-ethyl-2,3,4,5-tetrahydropyridazin-3-on;
mit Hexanoylchlorid:
2-(4-Hexanoylaminobenzyl)-6-(3,4-dimethoxyphenyl)-5-ethyl-2,3,4,5-tetrahydropyridazin-3-on;
mit Pentafluorpropionylchlorid:
2-(4-Pentafluorpropionylaminobenzyl)-6-(3,4-dimethoxyphenyl)-5-ethyl-2,3,4,5-tetrahydropyridazin-3-on.

Analog erhält man durch Umsetzung von 2-(4-Aminobenzyl)-6-(3-ethoxy-4-methoxyphenyl)-5-ethyl-2,3,4,5-tetrahydro-pyridazin-3-on
mit Acetylchlorid:
2-(4-Acetamidobenzyl)-6-(3-ethoxy-4-methoxyphenyl)-5-ethyl-2,3,4,5-tetrahydropyridazin-3-on;
mit Trifluoracetylchlorid:
2-(4-Trifluoracetamidobenzyl)-6-(3-ethoxy-4-methoxyphenyl)-5-ethyl-2,3,4,5-tetrahydropyridazin-3-on;
mit Methylsulfonylchlorid:
2-(4-Methylsulfonamidobenzyl)-6-(3-ethoxy-4-methoxyphenyl)-5-ethyl-2,3,4,5-tetrahydropyridazin-3-on;
mit Propionylchlorid:
2-(4-Propionyiaminobenzyl)-6-(3-ethoxy-4-methoxyphenyl)-5-ethyl-2,3,4,5-tetrahydropyridazin-3-on;
mit Butyrylchlorid:
2-(4-Butyrylaminobenzyl)-6-(3-ethoxy-4-methoxyphenyl)-5-ethyl-2,3,4,5-tetrahydropyridazin-3-on;
mit Isobutyrylchlorid:
2-(4-lsobutyrylaminobenzyl)-6-(3-ethoxy-4-methoxyphenyl)-5-ethyl-2,3,4,5-tetrahydropyridazin-3-on;
mit Chlorameisensäuremethylester:
2-(4-Methoxycarbonylaminobenzyl)-6-(3-ethoxy-4-methoxyphenyl)-5-ethyl-2,3,4,5-tetrahydropyridazin-3-on;
mit Pivalylchlorid:
2-(4-Pivalylaminobenzyl)-6-(3-ethoxy-4-methoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-ethoxy-4-methoxyphenyl)-5-ethyl-2,3,4,5-tetrahydropyridazin-3-on;
mit Chlorameisensäureethylester:
2-(4-Ethoxycarbonylaminobenzyl)-6-(3-ethoxy-4-methoxyphenyl)-5-ethyl-2,3,4,5-tetrahydropyridazin-3-on;
mit Methoxalyichlorid:
2-(4-Methoxalylaminobenzyl)-6-(3-ethoxy-4-methoxyphenyl)-5-ethyl-2,3,4,5-tetrahydropyridazin-3-on;
mit Chlorformamid:
2-(4-Ureidobenzyl)-6-(3-ethoxy-4-methoxyphenyl)-5-ethyl-2,3,4,5-tetrahydropyridazin-3-on;
mit Pentanoylchlorid:
2-(4-Pentanoylaminobenzyl)-6-(3-ethoxy-4-methoxyphenyl)-5-ethyl-2,3,4,5-tetrahydropyridazin-3-on;
mit Hexanoylchlorid:
2-(4-Hexanoylaminobenzyl)-6-(3-ethoxy-4-methoxyphenyl)-5-ethyl-2,3,4,5-tetrahydropyridazin-3-on;
mit Pentafluorpropionylchlorid:
2-(4-Pentafluorpropionylaminobenzyl)-6-(3-ethoxy-4-methoxyphenyl)-5-ethyl-2,3,4,5-tetrahydropyridazin-3-on.

Analog erhält man durch Umsetzung von 2-(4-Aminobenzyl)-6-(3-cyclopentyloxy-4-methoxyphenyl)-5-ethyl-2,3,4,5-tetrahydro-pyridazin-3-on
mit Acetylchlorid:
2-(4-Acetamidobenzyl)-6-(3-cyclopentyloxy-4-methoxyphenyl)-5-ethyl-2,3,4,5-tetrahydropyridazin-3-on;
mit Trifluoracetylchlorid:
2-(4-Trifluoracetamidobenzyl)-6-(3-cyclopentyloxy-4-methoxyphenyl)-5-ethyl-2,3,4,5-tetrahydropyridazin-3-on, F. 162°;
mit Methylsulfonylchlorid:
2-(4-Methylsulfonamidobenzyl)-6-(3-cyclopentyloxy-4-methoxyphenyl)-5-ethyl-2,3,4,5-tetrahydropyridazin-3-on;
mit Propionylchlorid:
2-(4-Propionylaminobenzyl)-6-(3-cyclopentyloxy-4-methoxyphenyl)-5-ethyl-2,3,4,5-tetrahydropyridazin-3-on, F. 69°;
mit 2,2-Dimethylpropionylchlorid:
2-(4-tert.-Butylcarbonylaminobenzyl)-6-(3-cyclopentyloxy-4-methoxyphenyl)-5-ethyl-2,3,4,5-tetrahydropyridazin-3-on;
mit Butyrylchlorid:
2-(4-Butyrylaminobenzyl)-6-(3-cyclopentyloxy-4-methoxyphenyl)-5-ethyl-2,3,4,5-tetrahydropyridazin-3-on;
mit Isobutyrylchlorid:
2-(4-lsobutyrylaminobenzyl)-6-(3-cyclopentyloxy-4-methoxyphenyl)-5-ethyl-2,3,4,5-tetrahydropyridazin-3-on;
mit Chlorameisensäuremethylester:
2-(4-Methoxycarbonylaminobenzyl)-6-(3-cyclopentyloxy-4-methoxyphenyl)-5-ethyl-2,3,4,5-tetrahydropyridazin-3-on;
mit Pivalylchlorid:
2-(4-Pivalylaminobenzyl)-6-(3-cyclopentyloxy-4-methoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-cyclopentyloxy-4-methoxyphenyl)-5-ethyl-2,3,4,5-tetrahydropyridazin-3-on;
mit Chlorameisensäureethylester:
2-(4-Ethoxycarbonylaminobenzyl)-6-(3-cyclopentyloxy-4-methoxyphenyl)-5-ethyl-2,3,4,5-tetrahydropyridazin-3-on, F. 73°;
mit Methoxalylchlorid:
2-(4-Methoxalylaminobenzyl)-6-(3-cyclopentyloxy-4-methoxyphenyl)-5-ethyl-2,3,4,5-tetrahydropyridazin-3-on;
mit Chlorformamid:
2-(4-Ureidobenzyl)-6-(3-cyclopentyloxy-4-methoxyphenyl)-5-ethyl-2,3,4,5-tetrahydropyridazin-3-on;
mit Pentanoylchlorid:
2-(4-Pentanoylaminobenzyl)-6-(3-cyclopentyloxy-4-methoxyphenyl)-5-ethyl-2,3,4,5-tetrahydropyridazin-3-on;
mit Hexanoylchlorid:
2-(4-Hexanoylaminobenzyl)-6-(3-cyclopentyloxy-4-methoxyphenyl)-5-ethyl-2,3,4,5-tetrahydropyridazin-3-on;
mit Pentafluorpropionylchlorid:
2-(4-Pentafluorpropionylamlnobenzyl)-6-(3-cyclopentyloxy-4-methoxyphenyl)-5-ethyl-2,3,4,5-tetrahydropyridazin-3-on.

Analog erhält man durch Umsetzung von 2-(4-Aminophenethyl)-6-(3,4-dimethoxyphenyl)-5-ethyl-2,3,4,5-tetrahydro-pyridazin-3-on
mit Acetylchlorid:
2-(4-Acetamidophenethyl)-6-(3,4-dimethoxyphenyl)-5-ethyl-2,3,4,5-tetrahydropyridazin-3-on;
mit Trifluoracetylchlorid:
2-(4-Trifluoracetamidophenethyl)-6-(3,4-dimethoxyphenyl)-5-ethyl-2,3,4,5-tetrahydropyridazin-3-on;
mit Methylsulfonylchlorid:
2-(4-Methylsulfonamidophenethyl)-6-(3,4-dimethoxyphenyl)-5-ethyl-2,3,4,5-tetrahydropyridazin-3-on;
mit Propionylchlorid:
2-(4-Propionylaminophenethyl)-6-(3,4-dimethoxyphenyl)-5-ethyl-2,3,4,5-tetrahyd ropyridazin-3-on;
mit Butyrylchlorid:
2-(4-Butyrylaminophenethyl)-6-(3,4-dimethoxyphenyl)-5-ethyl-2,3,4,5-tetrahydropyridazin-3-on;
mit Isobutyrylchlorid:
2-(4-lsobutyrylaminophenethyl)-6-(3,4-dimethoxyphenyl)-5-ethyl-2,3,4,5-tetrahydropyridazin-3-on;
mit Chlorameisensäuremethylester:
2-(4-Methoxycarbonylaminophenethyl)-6-(3,4-dimethoxyphenyl)-5-ethyl-2,3,4,5-tetrahydropyridazin-3-on;
mit Pivalylchlorid:
2-(4-Pivalylaminophenethyl)-6-(3,4-dimethoxyphenyl)-5-ethyl-2,3,4,5-tetrahydropyridazin-3-on;
mit Cyclopentancarbonsäurechlorid:
2-(4-Cyclopentylcarbamoylphenethyl)-6-(3,4-dimethoxyphenyl)-5-ethyl-2,3,4,5-tetrahydropyridazin-3-on;
mit Chlorameisensäureethylester
2-(4-Ethoxycarbonylaminophenethyl)-6-(3,4-dimethoxyphenyl)-5-ethyl-2,3,4,5-tetrahydropyridazin-3-on;
mit Methoxalylchlorid:
2-(4-Methoxalylaminophenethyl)-6-(3,4-dimethoxyphenyl)-5-ethyl-2,3,4,5-tetrahydropyridazin-3-on;
mit Chlorformamid:
2-(4-Ureidophenethyl)-6-(3,4-dimethoxyphenyl)-5-ethyl-2,3,4,5-tetrahydropyridazin-3-on;
mit Pentanoylchlorid:
2-(4-Pentanoylaminophenethyl)-6-(3,4-dimethoxyphenyl)-5-ethyl-2,3,4,5-tetrahydropyridazin-3-on;
mit Hexanoylchlorid:
2-(4-Hexanoylaminophenethyl)-6-(3,4-dimethoxyphenyl)-5-ethyl-2,3,4,5-tetrahydropyridazin-3-on;
mit Pentafluorpropionylchlorid:
2-(4-Pentafluorpropionylaminophenethyl)-6-(3,4-dimethoxyphenyl)-5-ethyl-2,3,4,5-tetrahydropyridazin-3-on.

Analog erhält man durch Umsetzung von 2-(3-Aminobenzyl)-6-(3,4-dimethoxyphenyl)-5-ethyl-2,3,4,5-tetrahydro-pyridazin-3-on
mit Acetylchlorid:
2-(3-Acetamidobenzyl)-6-(3,4-dimethoxyphenyl)-5-ethyl-2,3,4,5-tetrahydropyridazin-3-on;
mit Trifluoracetylchlorid:
2-(3-Trifluoracetamidobenzyl)-6-(3,4-dimethoxyphenyl)-5-ethyl-2,3,4,5-tetrahydropyridazin-3-on, F. 142°;
mit Methylsulfonylchlorid:
2-(3-Methylsulfonamidobenzyl)-6-(3,4-dimethoxyphenyl)-5-ethyl-2,3,4,5-tetrahydropyridazin-3-on;
mit Propionylchlorid:
2-(3-Propionylaminobenzyl)-6-(3,4-dimethoxyphenyl)-5-ethyl-2,3,4,5-tetrahydropyridazin-3-on, F. 126°;
mit Butyrylchlorid:
2-(3-Butyrylaminobenzyl)-6-(3,4-dimethoxyphenyl)-5-ethyl-2,3,4,5-tetrahydropyridazin-3-on;
mit Isobutyrylchlorid:
2-(3-lsobutyrylaminobenzyl)-6-(3,4-dimethoxyphenyl)-5-ethyl-2,3,4,5-tetrahydropyridazin-3-on;
mit Chlorameisensäuremethylester:
2-(3-Methoxycarbonylaminobenzyl)-6-(3,4-dimethoxyphenyl)-5-ethyl-2,3,4,5-tetrahydropyridazin-3-on;
mit Pivalylchlorid:
2-(3-Pivalylaminobenzyl)-6-(3,4-dimethoxyphenyl)-5-ethyl-2,3,4,5-tetrahydropyridazin-3-on;
mit Cyclopentancarbonsäurechlorid:
2-(3-Cyclopentylcarbamoylbenzyl)-6-(3,4-dimethoxyphenyl)-5-ethyl-2,3,4,5-tetrahydropyridazin-3-on;
mit Chlorameisensäureethylester:
2-(3-Ethoxycarbonylaminobenzyl)-6-(3,4-dimethoxyphenyl)-5-ethyl-2,3,4,5-tetrahydropyridazin-3-on, F. 54°;
mit Methoxalylchlorid:
2-(3-Methoxalylaminobenzyl)-6-(3,4-dimethoxyphenyl)-5-ethyl-2,3,4,5-tetrahydropyridazin-3-on;
mit Chlorformamid:
2-(3-Ureidobenzyl)-6-(3,4-dimethoxyphenyl)-5-ethyl-2,3,4,5-tetrahydropyridazin-3-on;
mit Pentanoylchlorid:
2-(3-Pentanoylaminobenzyl)-6-(3,4-dimethoxyphenyl)-5-ethyl-2,3,4,5-tetrahyd ropyridazin-3-on;
mit Hexanoylchlorid:
2-(3-Hexanoylaminobenzyl)-6-(3,4-dimethoxyphenyl)-5-ethyl-2,3,4,5-tetrahydropyridazin-3-on;
mit Pentafluorpropionylchlorid:
2-(3-Pentafluorpropionylaminobenzyl)-6-(3,4-dimethoxyphenyl)-5-ethyl-2,3,4,5-tetrahydropyridazin-3-on.

Analog erhält man durch Umsetzung von 2-(4-Aminobenzyl)-6-(3-fluormethoxy-4-methoxyphenyl)-5-ethyl-2,3,4,5-tetrahydro-pyridazin-3-on
mit Acetylchlorid:
2-(4-Acetamidobenzyl)-6-(3-fluormethoxy-4-methoxyphenyl)-5-ethyl-2,3,4,5-tetrahydropyridazin-3-on;
mit Trifluoracetylchlorid:
2-(4-Trifluoracetamidobenzyl)-6-(3-fluormethoxy-4-methoxyphenyl)-5-ethyl-2,3,4,5-tetrahydropyridazin-3-on;
mit Methylsulfonylchlorid:
2-(4-Methylsulfonamidobenzyl)-6-(3-fluormethoxy-4-methoxyphenyl)-5-ethyl-2,3,4,5-tetrahydropyridazin-3-on;
mit Propionylchlorid:
2-(4-Propionylaminobenzyl)-6-(3-fluormethoxy-4-methoxyphenyl)-5-ethyl-2,3,4,5-tetrahydropyridazin-3-on;
mit Butyrylchlorid:
2-(4-Butyrylaminobenzyl)-6-(3-fluormethoxy-4-methoxyphenyl)-5-ethyl-2,3,4,5-tetrahydropyridazin-3-on;
mit lsobutyrylchlorid:
2-(4-lsobutyrylaminobenzyl)-6-(3-fluormethoxy-4-methoxyphenyl)-5-ethyl-2,3,4,5-tetrahydropyridazin-3-on;
mit Chlorameisensäuremethylester:
2-(4-Methoxycarbonylaminobenzyl)-6-(3-fluormethoxy-4-methoxyphenyl)-5'-ethyl-2,3,4,5-tetrahydropyridazin-3-on;
mit Pivalylchlorid:
2-(4-Pivalylaminobenzyl)-6-(3-fluormethoxy-4-methoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-fluormethoxy-4-methoxyphenyl)-5-ethyl-2,3,4,5-tetrahydropyridazin-3-on;
mit Chlorameisensäureethylester:
2-(4-Ethoxycarbonylaminobenzyl)-6-(3-fluormethoxy-4-methoxyphenyl)-5-ethyl-2,3,4,5-tetrahydropyridazin-3-on;
mit Methoxalylchlorid:
2-(4-Methoxalylaminobenzyl)-6-(3-fluormethoxy-4-methoxyphenyl)-5-ethyl-2,3,4,5-tetrahydropyridazin-3-on;
mit Chlorformamid:
2-(4-Ureidobenzyl)-6-(3-fluormethoxy-4-methoxyphenyl)-5-ethyl-2,3,4,5-tetrahydropyridazin-3-on;
mit Pentanoylchlorid:
2-(4-Pentanoylaminobenzyl)-6-(3-fluormethoxy-4-methoxyphenyl)-5-ethyl-2,3'4,5-tetrahydropyridazin-3-on;
mit Hexanoylchlorid:
2-(4-Hexanoylaminobenzyl)-6-(3-fluormethoxy-4-methoxyphenyl)-5-ethyl-2,3,4,5-tetrahydropyridazin-3-on;
mit Pentafluorpropionylchlorid:
2-(4-Pentafluorpropionylaminobenzyl)-6-(3-fluormethoxy-4-methoxyphenyl)-5-ethyl-2,3,4,5-tetrahydropyridazin-3-on.

Analog erhält man durch Umsetzung von 2-(4-Aminobenzyl)-6-(3-difluormethoxy-4-methoxyphenyl)-5-ethyl-2,3,4,5-tetrahydro-pyridazin-3-on
mit Acetylchlorid:
2-(4-Acetamidobenzyl)-6-(3-difluormethoxy-4-methoxyphenyl)-5-ethyl-2,3,4,5-tetrahydropyridazin-3-on;
mit Trifluoracetylchlorid:
2-(4-Trifluoracetamidobenzyl)-6-(3-difluormethoxy-4-methoxyphenyl)-5-ethyl-2,3,4,5-tetrahydropyridazin-3-on;
mit Methylsulfonylchlorid:
2-(4-Methylsulfonamidobenzyl)-6-(3-difluormethoxy-4-methoxyphenyl)-5-ethyl-2,3,4,5-tetrahydropyridazin-3-on;
mit Propionylchlorid:
2-(4-Propionylaminobenzyl)-6-(3-difluormethoxy-4-methoxyphenyl)-5-ethyl-2,3,4,5-tetrahydropyridazin-3-on;
mit Butyrylchlorid:
2-(4-Butyrylaminobenzyl)-6-(3-difluormethoxy-4-methoxyphenyl)-5-ethyl-2,3,4,5-tetrahydropyridazin-3-on;
mit lsobutyrylchlorid:
2-(4-Isobutyrylaminobenzyl)-6-(3-difluormethoxy-4-methoxyphenyl)-5-ethyl-2,3,4,5-tetrahydropyridazin-3-on;
mit Chlorameisensäuremethylester:
2-(4-Methoxycarbonylaminobenzyl)-6-(3-difluormethoxy-4-methoxyphenyl)-5-ethyl-2,3,4,5-tetrahydropyridazin-3-on;
mit Pivalylchlorid:
2-(4-Pivalylaminobenzyl)-6-(3-difluormethoxy-4-methoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-difluormethoxy-4-methoxyphenyl)-5-ethyl-2,3,4,5-tetrahydropyridazin-3-on;
mit Chlorameisensäureethylester:
2-(4-Ethoxycarbonylaminobenzyl)-6-(3-difluormethoxy-4-methoxyphenyl)-5-ethyl-2,3,4,5-tetrahydropyridazin-3-on;
mit Methoxalylchlorid:
2-(4-Methoxalylaminobenzyl)-6-(3-difluormethoxy-4-methoxyphenyl)-5-ethyl-2,3,4,5-tetrahydropyridazin-3-on;
mit Chlorformamid:
2-(4-Ureidobenzyl)-6-(3-difluormethoxy-4-methoxyphenyl)-5-ethyl-2,3,4,5-tetrahydropyridazin-3-on;
mit Pentanoylchlorid:
2-(4-Pentanoylaminobenzyl)-6-(3-difluormethoxy-4-methoxyphenyl)-5-ethyl-2,3,4,5-tetrahydropyridazin-3-on;
mit Hexanoylchlorid:
2-(4-Hexanoylaminobenzyl)-6-(3-difluormethoxy-4-methoxyphenyl)-5-ethyl-2,3,4,5-tetrahydropyridazin-3-on;
mit Pentafluorpropionylchlorid:
2-(4-Pentafluorpropionylaminobenzyl)-6-(3-difluormethoxy-4-methoxyphenyl)-5-ethyl-2,3,4,5-tetrahydropyridazin-3-on.

Analog erhält man durch Umsetzung von 2-(4-Aminobenzyl)-6-(3-trifluormethoxy-4-methoxyphenyl)-5-ethyl-2,3,4,5-tetrahydro-pyridazin-3-on
mit Acetylchlorid:
2-(4-Acetamidobenzyl)-6-(3-trifluorrnethoxy-4-methoxyphenyl)-5-ethyl-2,3,4,5-tetrahydropyridazin-3-on;
mit Trifluoracetylchlorid:
2-(4-Trifluoracetamidobenzyl)-6-(3-trifluormethoxy-4-methoxyphenyl)-5-ethyl-2,3,4,5-tetrahydropyridazin-3-on;
mit Methylsulfonylchlorid:
2-(4-Methylsulfonamidobenzyl)-6-(3-trifluormethoxy-4-methoxyphenyl)-5-ethyl-2,3,4,5-tetrahydropyridazin-3-on;
mit Propionylchlorid:
2-(4-Propionylaminobenzyl)-6-(3-trifluormethoxy-4-methoxyphenyl)-5-ethyl-2,3,4,5-tetrahydropyridazin-3-on;
mit Butyrylchlorid:
2-(4-Butyrylaminobenzyl)-6-(3-trifluormethoxy-4-methoxyphenyl)-5-ethyl-2,3,4,5-tetrahydropyridazin-3-on;
mit Isobutyrylchlorid:
2-(4-lsobutyrylaminobenzyl)-6-(3-trifluormethoxy-4-methoxyphenyl)-5-ethyl-2,3,4,5-tetrahydropyridazin-3-on;
mit Chlorameisensäuremethylester:
2-(4-Methoxycarbonylaminobenzyl)-6-(3-trifluormethoxy-4-methoxyphenyl)-5-ethyl-2,3,4,5-tetrahydropyridazin-3-on;
mit Pivalylchlorid:
2-(4-Pivalylaminobenzyl)-6-(3-trifluormethoxy-4-methoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-trifluormethoxy-4-methoxyphenyl)-5-ethyl-2,3,4,5-tetrahydropyridazin-3-on;
mit Chlorameisensäureethylester:
2-(4-Ethoxycarbonylaminobenzyl)-6-(3-trifluormethoxy-4-methoxyphenyl)-5-ethyl-2,3,4,5-tetrahydropyridazin-3-on;
mit Methoxalylchlorid:
2-(4-Methoxalylaminobenzyl)-6-(3-trifluormethoxy-4-methoxyphenyl)-5-ethyl-2,3,4,5-tetrahydropyridazin-3-on;
mit Chlorformamid:
2-(4-Ureidobenzyl)-6-(3-trifluormethoxy-4-methoxyphenyl)-5-ethyl-2,3,4,5-tetrahydropyridazin-3-on;
mit Pentanoylchlorid:
2-(4-Pentanoylaminobenzyl)-6-(3-trifluormethoxy-4-methoxyphenyl)-5-ethyl-2,3,4,5-tetrahydropyridazin-3-on;
mit Hexanoylchlorid:
2-(4-Hexanoylaminobenzyl)-6-(3-trifluormethoxy-4-methoxyphenyl)-5-ethyl-2,3,4,5-tetrahydropyridazin-3-on;
mit Pentafluorpropionylchlorid:
2-(4-Pentafluorpropionylaminobenzyl)-6-(3-trifluormethoxy-4-methoxyphenyl)-5-ethyl-2,3,4,5-tetrahydropyridazin-3-on.

Analog erhält man durch Umsetzung von 2-(4-Aminobenzyl)-6-(3-methoxy-4-fluormethoxyphenyl)-5-ethyl-2,3,4,5-tetrahydro-pyridazin-3-on
mit Acetylchlorid:
2-(4-Acetamidobenzyl)-6-(3-methoxy-4-fluormethoxyphenyl)-5-ethyl-2,3,4,5-tetrahydropyridazin-3-on;
mit Trifluoracetylchlorid:
2-(4-Trifluoracetamidobenzyl)-6-(3-methoxy-4-fluormethoxyphenyl)-5-ethyl-2,3,4,5-tetrahydropyridazin-3-on;
mit Methylsulfonylchlorid:
2-(4-Methylsulfonamidobenzyl)-6-(3-methoxy-4-fluormethoxyphenyl)-5-ethyl-2,3,4,5-tetrahydropyridazin-3-on;
mit Propionylchlorid:
2-(4-Propionylaminobenzyl)-6-(3-methoxy-4-fluormethoxyphenyl)-5-ethyl-2,3,4,5-tetrahydropyridazin-3-on;
mit Butyrylchlorid:
2-(4-Butyrylaminobenzyl)-6-(3-methoxy-4-fluormethoxyphenyl)-5-ethyl-2,3,4,5-tetrahydropyridazin-3-on;
mit Isobutyrylchlorid:
2-(4-lsobutyrylaminobenzyl)-6-(3-methoxy-4-fluormethoxyphenyl)-5-ethyl-2,3,4,5-tetrahydropyridazin-3-on;
mit Chlorameisensäuremethylester:
2-(4-Methoxycarbonylaminobenzyl)-6-(3-methoxy-4-fluormethoxyphenyl)-5-ethyl-2,3,4,5-tetrahydropyridazin-3-on;
mit Pivalylchlorid:
2-(4-Pivalylaminobenzyl)-6-(3-methoxy-4-fluormethoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-methoxy-4-fluormethoxyphenyl)-5-ethyl-2,3,4,5-tetrahydropyridazin-3-on;
mit Chlorameisensäureethylester:
2-(4-Ethoxycarbonylaminobenzyl)-6-(3-methoxy-4-fluormethoxyphenyl)-5-ethyl-2,3,4,5-tetrahydropyridazin-3-on;
mit Methoxalylchlorid:
2-(4-Methoxalylaminobenzyl)-6-(3-methoxy-4-fluormethoxyphenyl)-5-ethyl-2,3,4,5-tetrahydropyridazin-3-on;
mit Chlorformamid:
2-(4-Ureidobenzyl)-6-(3-methoxy-4-fluormethoxyphenyl)-5-ethyl-2,3,4,5-tetrahydropyridazin-3-on;
mit Pentanoylchlorid:
2-(4-Pentanoylaminobenzyl)-6-(3-methoxy-4-fluormethoxyphenyl)-5-ethyl-2,3,4,5-tetrahydropyridazin-3-on;
mit Hexanoylchlorid:
2-(4-Hexanoylaminobenzyl)-6-(3-methoxy-4-fluormethoxyphenyl)-5-ethyl-2,3,4,5-tetrahydropyridazin-3-on;
mit Pentafluorpropionylchlorid:
2-(4-Pentafluorpropionylaminobenzyl)-6-(3-methoxy-4-fluormethoxyphenyl)-5-ethyl-2,3,4,5-tetrahydropyridazin-3-on.

Analog erhält man durch Umsetzung von 2-(4-Aminobenzyl)-6-(3-methoxy-4-difluormethoxyphenyl)-5-ethyl-2,3,4,5-tetrahydro-pyridazin-3-on
mit Acetylchlorid:
2-(4-Acetamidobenzyl)-6-(3-methoxy-4-difluormethoxyphenyl)-5-ethyl-2,3,4,5-tetrahydropyridazin-3-on;
mit Trifluoracetylchlorid:
2-(4-Trifluoracetamidobenzyl)-6-(3-methoxy-4-difluormethoxyphenyl)-5-ethyl-2,3,4,5-tetrahydropyridazin-3-on;
mit Methylsulfonylchlorid:
2-(4-Methylsulfonamidobenzyl)-6-(3-methoxy-4-difluormethoxyphenyl)-5-ethyl-2,3,4,5-tetrahydropyridazin-3-on;
mit Propionylchlorid:
2-(4-Propionylaminobenzyl)-6-(3-methoxy-4-difluormethoxyphenyl)-5-ethyl-2,3,4,5-tetrahydropyridazin-3-on;
mit Butyrylchlorid:
2-(4-Butyrylaminobenzyl)-6-(3-methoxy-4-difluormethoxyphenyl)-5-ethyl-2,3,4,5-tetrahydropyridazin-3-on;
mit Isobutyrylchlorid:
2-(4-Isobutyrylaminobenzyl)-6-(3-methoxy-4-difluormethoxyphenyl)-5-ethyl-2,3,4,5-tetrahydropyridazin-3-on;
mit Chlorameisensäuremethylester:
2-(4-Methoxycarbonylaminobenzyl)-6-(3-methoxy-4-difluormethoxyphenyl)-5-ethyl-2,3,4,5-tetrahydropyridazin-3-on;
mit Pivalylchlorid:
2-(4-Pivalylaminobenzyl)-6-(3-methoxy-4-difluormethoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-methoxy-4-difluormethoxyphenyl)-5-ethyl-2,3,4,5-tetrahydropyridazin-3-on;
mit Chlorameisensäureethylester
2-(4-Ethoxycarbonylaminobenzyl)-6-(3-methoxy-4-difluormethoxyphenyl)-5-ethyl-2,3,4,5-tetrahydropyridazin-3-on;
mit Methoxalylchlorid:
2-(4-Methoxalylaminobenzyl)-6-(3-methoxy-4-difluormethoxyphenyl)-5-ethyl-2,3,4,5-tetrahydropyridazin-3-on;
mit Chlorformamid:
2-(4-Ureidobenzyl)-6-(3-methoxy-4-difluormethoxyphenyl)-5-ethyl-2,3,4,5-tetrahydropyridazin-3-on;
mit Pentanoylchlorid:
2-(4-Pentanoylaminobenzyl)-6-(3-methoxy-4-difluormethoxyphenyl)-5-ethyl-2,3,4,5-tetrahydropyridazin-3-on;
mit Hexanoylchlorid:
2-(4-Hexanoylaminobenzyl)-6-(3-methoxy-4-difluormethoxyphenyl)-5-ethyl-2,3,4,5-tetrahydropyridazin-3-on;
mit Pentafluorpropionylchlorid:
2-(4-Pentafluorpropionylaminobenzyl)-6-(3-methoxy-4-difluormethoxyphenyl)-5-ethyl-2,3,4,5-tetrahydropyridazin-3-on.

Analog erhält man durch Umsetzung von 2-(4-Aminobenzyl)-6-(3-methoxy-4-trifluormethoxyphenyl)-5-ethyl-2,3,4,5-tetrahydro-pyridazin-3-on
mit Acetylchlorid:
2-(4-Acetamidobenzyl)-6-(3-methoxy-4-trifluormethoxyphenyl)-5-ethyl-2,3,4,5-tetrahydropyridazin-3-on;
mit Trifluoracetylchlorid:
2-(4-Trifluoracetamidobenzyl)-6-(3-methoxy-4-trifluormethoxyphenyl)-5-ethyl-2,3,4,5-tetrahydropyridazin-3-on;
mit Methylsulfonylchlorid:
2-(4-Methylsulfonamidobenzyl)-6-(3-methoxy-4-trifluormethoxyphenyl)-5-ethyl-2,3,4,5-tetrahydropyridazin-3-on;
mit Propionylchlorid:
2-(4-Propionylaminobenzyl)-6-(3-methoxy-4-trifluormethoxyphenyl)-5-ethyl-2,3,4,5-tetrahydropyridazin-3-on;
mit Butyrylchlorid:
2-(4-Butyrylaminobenzyl)-6-(3-methoxy-4-trifluormethoxyphenyl)-5-ethyl-2,3,4,5-tetrahydropyridazin-3-on;
mit Isobutyrylchlorid:
2-(4-lsobutyrylaminobenzyl)-6-(3-methoxy-4-trifluormethoxyphenyl)-5-ethyl-2,3,4,5-tetrahydropyridazin-3-on;
mit Chlorameisensäuremethylester:
2-(4-Methoxycarbonylaminobenzyl)-6-(3-methoxy-4-trifluormethoxyphenyl)-5-ethyl-2,3,4,5-tetrahydropyridazin-3-on;
mit Pivalylehforid:
2-(4-Pivalylaminobenzyl)-6-(3-methoxy-4-trifluormethoxyphenyl)-5-ethyl-2,3,4,5-tetrahydropyridazin-3-on;
mit Cyclopentancarbonsäurechlorid:
2-(4-Cyclopentylcarbamoylbenzyl)-6-(3-methoxy-4-trifluormethoxyphenyl)-5-ethyl-2,3,4,5-tetrahydropyridazin-3-on;
mit Chlorameisensäureethylester:
2-(4-Ethoxycarbonylaminobenzyl)-6-(3-methoxy-4-trifluormethoxyphenyl)-5-ethyl-2,3,4,5-tetrahydropyridazin-3-on;
mit Methoxalylchlorid:
2-(4-Methoxalylaminobenzyl)-6-(3-methoxy-4-trifluormethoxyphenyl)-5-ethyl-2,3,4,5-tetrahydropyridazin-3-on;
mit Chlorformamid:
2-(4-Ureidobenzyl)-6-(3-methoxy-4-trifluormethoxyphenyl)-5-ethyl-2,3,4,5-tetrahydropyridazin-3-on;
mit Pentanoylchlorid:
2-(4-Pentanoylaminobenzyl)-6-(3-methoxy-4-trifluormethoxyphenyl)-5-ethyl-2,3,4,5-tetrahydropyridazin-3-on;
mit Hexanoylchlorid:
2-(4-Hexanoylaminobenzyl)-6-(3-methoxy-4-trifluormethoxyphenyl)-5-ethyl-2,3,4,5-tetrahydropyridazin-3-on;
mit Pentafluorpropionylchlorid:
2-(4-Pentafluorpropionylaminobenzyl)-6-(3-methoxy-4-trifluormethoxyphenyl)-5-ethyl-2,3,4,5-tetrahydropyridazin-3-on.

Analog erhält man durch Umsetzung von 2-(3-Aminobenzyl)-6-(3-cyclopentyloxy-4-methoxyphenyl)-5-ethyl-2,3,4,5-tetrahydro-pyridazin-3-on
mit Acetylchlorid:
2-(3-Acetamidobenzyl)-6-(3-cyclopentyloxy-4-methoxyphenyl)-5-ethyl-2,3,4,5-tetrahydropyridazin-3-on;
mit Trifluoracetylchlorid:
2-(3-Trifluoracetamidobenzyl)-6-(3-cyclopentyloxy-4-methoxyphenyl)-5-ethyl-2,3,4,5-tetrahydropyridazin-3-on, F. 70°;
mit Methylsulfonylchlorid:
2-(3-Methylsulfonamidobenzyl)-6-(3-cyclopentyloxy-4-methoxyphenyl)-5-ethyl-2,3,4,5-tetrahydropyridazin-3-on;
mit Propionylchlorid:
2-(3-Propionylaminobenzyl)-6-(3-cyclopentyloxy-4-methoxyphenyl)-5-ethyl-2,3,4,5-tetrahydropyridazin-3-on, F. 113°;
mit 2,2-Dimethylpropionylchlorid:
2-(3-tert.-Butylcarbonylaminobenzyl)-6-(3-cyclopentyloxy-4-methoxyphenyl)-5-ethyl-2,3,4,5-tetrahydropyridazin-3-on,
mit Butyrylchlorid:
2-(3-Butyrylaminobenzyl)-6-(3-cyclopentyloxy-4-methoxyphenyl)-5-ethyl-2,3,4,5-tetrahydropyridazin-3-on;
mit Isobutyrylchlorid:
2-(3-lsobutyrylaminobenzyl)-6-(3-cyclopentyloxy-4-methoxyphenyl)-5-ethyl-2,3,4,5-tetrahydropyridazin-3-on;
mit Chlorameisensäuremethylester:
2-(3-Methoxycarbonylaminobenzyl)-6-(3-cyclopentyloxy-4-methoxyphenyl)-5-ethyl-2,3,4,5-tetrahydropyridazin-3-on;
mit Pivalylchlorid:
2-(3-Pivalylaminobenzyl)-6-(3-cyclopentyloxy-4-methoxyphenyl)-5-ethyl-2,3,4,5-tetrahydropyridazin-3-on;
mit Cyclopentancarbonsäurechlorid:
2-(3-Cyclopentylcarbamoylbenzyl)-6-(3-cyclopentyloxy-4-methoxyphenyl)-5-ethyl-2,3,4,5-tetrahydropyridazin-3-on;
mit Chlorameisensäureethylester:
2-(3-Ethoxycarbonylaminobenzyl)-6-(3-cyclopentyloxy-4-methoxyphenyl)-5-ethyl-2,3,4,5-tetrahydropyridazin-3-on, F. 153°;
mit Methoxalylchlorid:
2-(3-Methoxalylaminobenzyl)-6-(3-cyclopentyloxy-4-methoxyphenyl)-5-ethyl-2,3,4,5-tetrahydropyridazin-3-on;
mit Chlorformamid:
2-(3-Ureidobenzyl)-6-(3-cyclopentyloxy-4-methoxyphenyl)-5-ethyl-2,3,4,5-tetrahydropyridazin-3-on;
mit Pentanoylchlorid:
2-(3-Pentanoylaminobenzyl)-6-(3-cyclopentyloxy-4-methoxyphenyl)-5-ethyl-2,3,4,5-tetrahydropyridazin-3-on;
mit Hexanoylchlorid:
2-(3-Hexanoylaminobenzyl)-6-(3-cyclopentyloxy-4-methoxyphenyl)-5-ethyl-2,3,4,5-tetrahydropyridazin-3-on;
mit Pentafluorpropionylchlorid:
2-(3-Pentafluorpropionylaminobenzyl)-6-(3-cyclopentyloxy-4-methoxyphenyl)-5-ethyl-2,3,4,5-tetrahydropyridazin-3-on.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 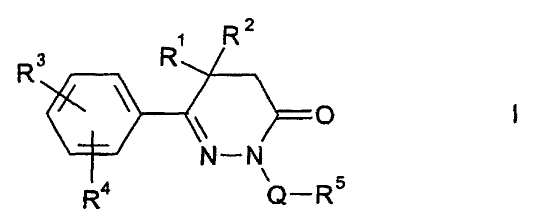 worin
R¹ H,
R² Methyl oder Ethyl,
R³ und R⁴ jeweils unabhängig voneinander -OH, -OR¹⁰, -S-R¹⁰, -SO-R¹⁰, -SO₂-R¹⁰, Hal, Methylendioxy, -NO₂, -NH₂, -NHR¹⁰ oder -NR¹⁰R¹¹,
R⁵ einen ein- oder zweifach durch R⁶ und/oder R⁷ substituierten Phenylrest,
Q fehlt oder Alkylen mit 1-6 C-Atomen,
R⁶ und R⁷ jeweils unabhängig voneinander -NH₂, -NR⁸R⁹, -NHR¹⁰, -NR¹⁰R¹¹, -NO₂, Hal, -CN, -OA, -COOH oder-COOA,
R⁸ und R⁹ jeweils unabhängig voneinander H, Acyl mit 1-8 C-Atomen, das durch 1-5 F- und/oder Cl-Atome substituiert sein kann, -COOA, -SO-A, -SO₂A,
-CONH₂, -CONHA, -CONA₂, -CO-COOH, -CO-COOA, -CO-CONH₂, -CO-CONHA oder -CO-CONA₂,
A Alkyl mit 1 bis 6 C-Atomen, das durch 1-5 F- und/oder Cl-Atome substituiert sein kann,
R¹⁰ und R¹¹ jeweils unabhängig voneinander A, Cycloalkyl mit 3-7 C-Atomen, Methylencycloalkyl mit 4-8 C-Atomen oder Alkenyl mit 2-8 C-Atomen
und
Hal F, Cl, Br oder I
bedeuten,
sowie deren physiologisch unbedenklichen Salze.

2. Ein Enantiomer einer Verbindung der Formel I gemäß Anspruch 1.

3. Verbindungen nach Anspruch 1
a) 2-(4-Trifluoracetamidobenzyl)-6-(3,4-dimethoxyphenyl)-5-ethyl-2,3,4,5-tetrahydro-pyridazin-3-on;
b) 2-(4-Ethoxycarbonylaminobenzyl)-6-(3,4-dimethoxyphenyl)-5-ethyl-2,3,4,5-tetrahydro-pyridazin-3-on;
c) 2-(4-Methoxycarbonylaminobenzyl)-6-(3,4-dimethoxyphenyl)-5-ethyl-2,3,4,5-tetrahydro-pyridazin-3-on;
d) 2-(4-Butyrylaminobenzyl)-6-(3,4-dimethoxyphenyl)-5-ethyl-2,3,4,5-tetrahydro-pyridazin-3-on;
e) 2-(4-Ethoxycarbonylaminobenzyl)-6-(3-ethoxy-4-methoxyphenyl)-5-ethyl-2,3,4,5-tetrahydro-pyridazin-3-on.

4. Verfahren zur Herstellung von Verbindungen der Formel I nach Anspruch 1 sowie deren Salzen, **dadurch gekennzeichnet, daß** man eine Verbindung der Formel II 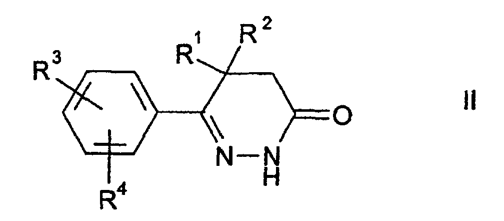 worin
R¹, R², R³ und R⁴ die in Anspruch 1 angegebenen Bedeutungen haben,
mit einer Verbindung der Formel III
R⁵-Q-X III
worin
R⁵ und Q die angegebenen Bedeutungen haben, und
X Cl, Br, OH oder eine reaktionsfähige veresterte OH-Gruppe bedeutet.
umsetzt,
oder daß man eine Verbindung der Formel IV 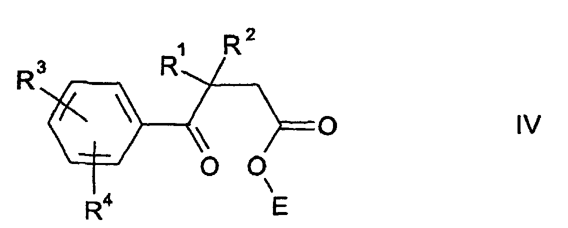
R¹, R², R³ und R⁴ die angegebenen Bedeutungen haben, und
E H oder Alkyl mit 1-4 C-Atomen bedeutet,
mit einer Verbindung der Formel V
H₂N-NH-Q-R⁵ V
worin
Q und R⁵ die angegebenen Bedeutungen haben,
umsetzt,
oder daß man in einer Verbindung der Formel I einen Rest R⁵ in einen anderen Rest R⁵ umwandelt, indem man eine Nitrogruppe reduziert, eine primäre oder eine sekundäre Aminogruppe alkyliert oder acyliert oder eine Cyangruppe hydrolysiert,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-X bzw. R⁴-X, worin R³, R⁴ und X die angegebenen Bedeutungen haben, umsetzt und/oder eine Base der Formel I durch Behandeln mit einer Säure in eines ihrer Salze umwandelt.

5. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

6. Pharmazeutische Zubereitung, **gekennzeichnet durch** einen Gehalt an mindestens einer Verbindung der Formel I nach Anspruch 1 und/oder einem ihrer physiologisch unbedenklichen Salze.

7. Verbindungen der Formel I nach Anspruch 1 und ihre physiologisch unbedenklichen Salze zur Bekämpfung von Krankheiten.

8. Arzneimittel der Formel I nach Anspruch 1 und ihre physiologisch unbedenklichen Salze als Phosphodiesterase IV-Hemmer.

9. Verwendung von Verbindungen der Formel I nach Anspruch 1 und/oder ihre physiologisch unbedenklichen Salze zur Herstellung eines Arzneimittels.

10. Verwendung nach Anspruch 9 zur Herstellung eines Arzneimittels zur Behandlung von asthmatischen, allergischen, entzündlichen und Autoimmunkrankheiten, Transplantatabstossungsreaktionen und Gedächtnisstörungen.

## Claims

1. Compounds of the formula I 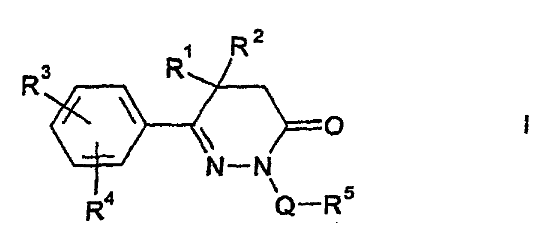 in which
R¹ is H,
R² is methyl or ethyl,
R³ and R⁴ are each, independently of one another, -OH, -OR¹⁰, -S-R¹⁰, -SO-R¹⁰, -SO₂-R¹⁰, Hal, methylenedioxy, -NO₂, -NH₂, -NHR¹⁰ or-NR¹⁰R¹¹,
R⁵ is a phenyl radical which is monosubstituted or disubstituted by R⁶ and/or R⁷,
Q is absent or is alkylene having 1-6 carbon atoms,
R⁶ and R⁷ are each, independently of one another, -NH₂, -NR⁸R⁹, -NHR¹⁰, -NR¹⁰R¹¹, -NO₂, Hal, -CN, -OA, -COOH or -COOA,
R⁸ and R⁹ are each, independently of one another, H, acyl having 1-8 carbon atoms, which may be substituted by 1-5 F and/or Cl atoms, -COOA, -SO-A, -SO₂A, -CONH₂, -CONHA, -CONA₂, -CO-COOH, -CO-COOA, -CO-CONH₂, -CO-CONHA or -CO-CONA₂,
A is alkyl having from 1 to 6 carbon atoms, which may be substituted by 1-5 F and/or Cl atoms,
R¹⁰ and R¹¹ are each, independently of one another, A, cycloalkyl having 3-7 carbon atoms, methylenecycloalkyl having 4-8 carbon atoms or alkenyl having 2-8 carbon atoms,
and
Hal is F, Cl, Br or I,
and physiologically acceptable salts thereof.

2. An enantiomer of a compound of the formula I according to Claim 1.

3. Compounds according to Claim 1
a) 2-(4-trifluoroacetamidobenzyl)-6-(3,4-dimethoxyphenyl)-5-ethyl-2,3,4,5-tetrahydropyridazin-3-one;
b) 2-(4-ethoxycarbonylaminobenzyl)-6-(3,4-dimethoxyphenyl)-5-ethyl-2,3,4,5-tetrahydropyridazin-3-one;
c) 2-(4-methoxycarbonylaminobenzyl)-6-(3,4-dimethoxyphenyl)-5-ethyl-2,3,4,5-tetrahydropyridazin-3-one;
d) 2-(4-butyrylaminobenzyl)-6-(3,4-dimethoxyphenyl)-5-ethyl-2,3,4,5-tetrahydropyridazin-3-one;
e) 2-(4-ethoxycarbonylaminobenzyl)-6-(3-ethoxy-4-methoxyphenyl)-5-ethyl-2,3,4,5-tetrahydropyridazin-3-one.

4. Process for the preparation of compounds of the formula I according to Claim 1 and salts thereof, **characterised in that** a compound of the formula II 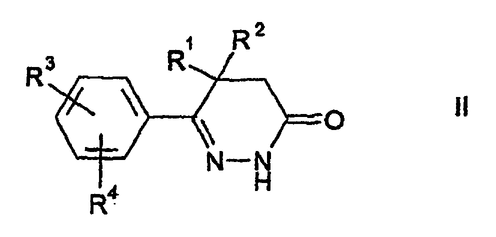 in which
R¹, R², R³ and R⁴ are as defined in Claim 1,
is reacted with a compound of the formula III
R⁵-Q-X III
in which
R⁵ and Q are as defined above, and
X is Cl, Br, OH or a reactive esterified OH group,
or **in that** a compound of the formula IV 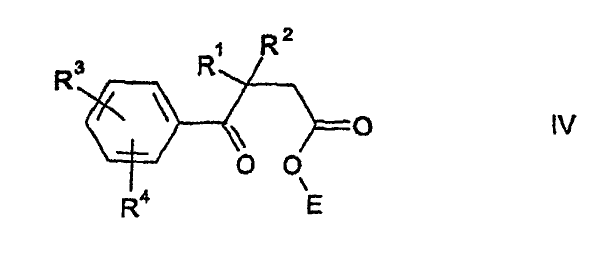 in which
R¹, R², R³ and R⁴ are as defined above, and
E is H or alkyl having 1-4 carbon atoms,
is reacted with a compound of the formula V
H₂N-NH-Q-R⁵ V
in which
Q and R⁵ are as defined above,
or **in that** a radical R⁵ in a compound of the formula I is converted into another radical R⁵ by reducing a nitro group, alkylating or acylating a primary or secondary amino group or hydrolysing a cyano group,
and/or **in that**, if desired, a compound which conforms to the formula I, but which contains one or two free OH groups instead of R³ and/or R⁴, is reacted with a compound of the formula R³-X or R⁴-X, in which R³, R⁴ and X are as defined above, and/or a base of the formula I is converted into one of its salts by treatment with an acid.

5. Process for the preparation of pharmaceutical preparations, **characterised in that** a compound of the formula I according to Claim 1 and/or one of its physiologically acceptable salts is converted into a suitable dosage form together with at least one solid, liquid or semiliquid excipient or adjuvant.

6. Pharmaceutical preparation, **characterised by** a content of at least one compound of the formula I according to Claim 1 and/or one of its physiologically acceptable salts.

7. Compounds of the formula I according to Claim 1 and physiologically acceptable salts thereof for combating diseases.

8. Medicament of the formula I according to Claim 1 and physiologically acceptable salts thereof as phosphodiesterase IV inhibitors.

9. Use of compounds of the formula I according to Claim 1 and/or physiologically acceptable salts thereof for the preparation of a medicament.

10. Use according to Claim 9 for the preparation of a medicament for the treatment of asthmatic, allergic, inflammatory and autoimmune diseases, transplant rejection reactions and memory disorders.

## Revendications

1. Composés répondant à la formule I 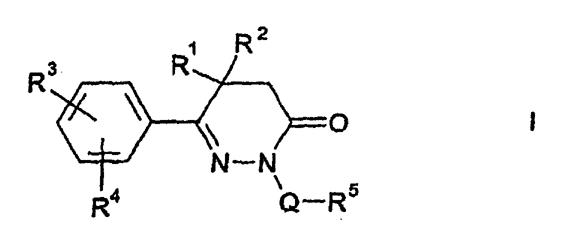 dans laquelle
R¹ représente H,
R² représente méthyle ou éthyle,
R³ et R⁴ représentent chacun, indépendamment l'un de l'autre, -OH, -OR¹⁰, -S-R¹⁰, -SO-R¹⁰, -SO₂-R¹⁰, Hal, méthylènedioxy, -NO₂, -NH₂, -NHR¹⁰ ou -NR¹⁰R¹¹,
R⁵ représente un radical phényle qui est monosubstitué ou disubstitué par R⁶ et/ou R⁷,
Q est absent ou représente alkylène ayant de 1 à 6 atomes de carbone,
R⁶ et R⁷ représentent chacun, indépendamment l'un de l'autre, -NH₂, -NR⁸R⁹, -NHR¹⁰, -NR¹⁰R¹¹, -NO₂, Hal, -CN, -OA, -COOH ou -COOA,
R⁸ et R⁹ représentent chacun, indépendamment l'un de l'autre, H, acyle ayant de 1 à 8 atomes de carbone, pouvant être substitué par de 1 à 5 atomes de F et/ou de Cl, -CODA, -SO-A, -SO₂A, -CONH₂, - CONHA, -CONA₂, -CO-COOH, -CO-COOA, -CO-CONH₂, -CO-CONHA ou -CO-CONA₂,
A représente alkyle ayant de 1 à 6 atomes de carbone, pouvant être substitué par de 1 à 5 atomes de F et/ou de Cl,
R¹⁰ et R¹¹ représentent chacun, indépendamment l'un de l'autre, A, cycloalkyle ayant de 3 à 7 atomes de carbone, méthylènecycloalkyle ayant de 4 à 8 atomes de carbone ou alkényle ayant de 2 à 8 atomes de carbone,
et
Hal représente F, Cl, Br ou I,
et les sels physiologiquement acceptables de ceux-ci.

2. Enantiomère d'un composé répondant à la formule I selon la revendication 1.

3. Composés selon la revendication 1
a) la 2-(4-trifluoroacétamidobenzyl)-6-(3,4-diméthoxyphényl)-5-éthyl-2,3,4,5-tétrahydropyridazin-3-one;
b) la 2-(4-éthoxycarbonylaminobenzyl)-6-(3,4-diméthoxyphényl)-5-éthyl-2,3,4,5-tétrahydropyridazin-3-one;
c) la 2-(4-méthoxycarbonylaminobenzyl)-6-(3,4-diméthoxyphényl)-5-éthyl-2,3,4,5-tétrahydropyridazin-3-one;
d) la 2-(4-butyrylaminobenzyl)-6-(3,4-diméthoxyphényl)-5-éthyl-2,3,4,5-tétrahydropyridazin-3-one;
e) la 2-(4-éthoxycarbonylaminobenzyl)-6-(3-éthoxy-4-méthoxyphényl)-5-éthyl-2,3,4,5-tétrahydropyridazin-3-one.

4. Procédé de préparation de composés répondant à la formule I selon la revendication 1 et de sels de ceux-ci, **caractérisé en ce qu'**un composé répondant à la formule II 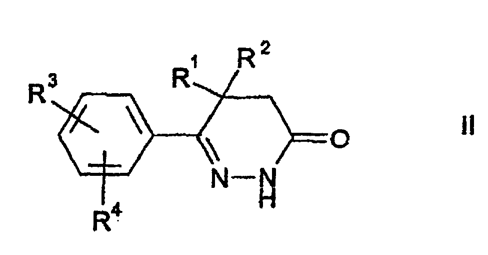 dans laquelle
R¹, R², R³ et R⁴ sont tels que définis selon la revendication 1,
est réagi avec un composé répondant à la formule III
R⁵-Q-X III
dans laquelle
R⁵ et Q sont tels que définis ci-dessus, et
X représente Cl, Br, OH ou un groupement OH estérifié réactif,
ou **en ce qu'**un composé répondant à la formule IV 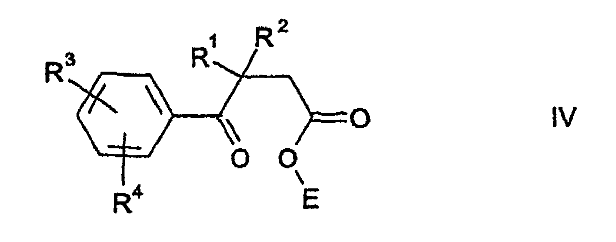 dans laquelle
R¹, R², R³ et R⁴ sont tels que définis ci-dessus, et
E représente H ou alkyle ayant de 1 à 4 atomes de carbone,
est réagi avec un composé répondant à la formule V
H₂N-NH-Q-R⁵ V
dans laquelle
Q et R⁵ sont tels que définis ci-dessus,
ou **en ce qu'**un radical R⁵ dans un composé répondant à la formule I est converti en un autre radical R⁵ par la réduction d'un groupement nitro, l'alkylation ou l'acylation d'un groupement amine primaire ou secondaire ou l'hydrolyse d'un groupement cyano,
et/ou **en ce que**, si on le désire, un composé conforme à la formule I, mais qui contient un ou deux groupements OH libres à la place de R³ et/ou de R⁴, est réagi avec un composé répondant à la formule R³-X ou R⁴-X, dans laquelle R³, R⁴ et X sont tels que définis ci-dessus, et/ou une base répondant à la formule I est convertie en l'un de ses sels par un traitement par un acide.

5. Procédé de préparation de préparations pharmaceutiques, **caractérisé en ce qu'**un composé répondant à la formule I selon la revendication 1 et/ou l'un de ses sels physiologiquement acceptables est mis sous une forme de dosage convenable conjointement avec au moins un excipient ou adjuvant solide, liquide ou semi-liquide.

6. Préparation pharmaceutique, **caractérisée par** une teneur en au moins un composé répondant à la formule I selon la revendication 1 et/ou en l'un de ses sels physiologiquement acceptables.

7. Composés répondant à la formule I selon la revendication 1 et les sels physiologiquement acceptables de ceux-ci pour lutter contre des maladies.

8. Médicament répondant à la formule I selon la revendication 1 et les sels physiologiquement acceptables de ceux-ci comme inhibiteurs de la phosphodiestérase IV.

9. Utilisation de composés répondant à la formule I selon la revendication 1 et/ou de sels physiologiquement acceptables de ceux-ci pour la préparation d'un médicament.

10. Utilisation selon la revendication 9 pour la préparation d'un médicament pour le traitement de maladies asthmatiques, allergiques, inflammatoires et auto-immunes, des réactions de rejet de greffon et des troubles de la mémoire.
